# EUROPEAN PATENT APPLICATION

(11) **EP 3 950 008 A1**
(43) Date of publication of application: **09.02.2022**
(21) Application number: 19922553.3
(22) Date of filing: 17.09.2019
(51) Int. Cl.: A61K 51/04, G01N 33/60

(54) **COMPOSITION FOR DIAGNOSIS OF FUNGAL INFECTION**

(30) Priority: 01.04.2019 KR 20190037711
(71) Applicant: Cncure Biotech Inc., Jeollanam-do 58128 (KR)
(72) Inventor: MIN, Jung-Joon, Gwangju 61461 (KR); KIM, Dong-Yeon, Hwasun-gun Jeollanam-do 58126 (KR); PYO, Ayoung, Gwangju 62003 (KR); KANG, Seung Ji, Gwangju 61160 (KR); KANG, Sae-Ryung, Gwangju 61706 (KR); KIM, Uh Jin, Gwangju 61664 (KR)
(74) Representative: Dehns
(86) International application number: PCT/KR2019/011963
(87) International publication number: WO 2020/204279

(57) **Abstract**

The present invention provides a composition for diagnosing fungal infection comprising 2-deoxy-2-[¹⁸F]fluorosorbitol ([¹⁸F]FDS) as an effective ingredient. The present invention makes it possible to quickly and effectively diagnose fungal infections and to acquire diagnostic images specialized for invasive fungal infections, which have been difficult to diagnose with conventional microbiological technologies and general imaging technologies,

## Description

### TECHNICAL FIELD

The present invention relates to a composition for diagnosing a fungal infection.

### BACKGROUND

In general, fungi reside in the human body or are widely distributed in the surrounding natural environment, such as soil, air, and water, and can often be harmful or beneficial to humans. Some fungi are common pathogens that cause disease in humans and animals, and whether or not to be infected depends on the host exposure status, such as the amount of infection, the route of invasion, and the host's immune status. Diseases caused by fungi are generally: first, hypersensitivity diseases caused by sensitization to specific fungal antigens, second, mycotoxicosis caused by mycotoxin produced by fungi, and third, mycosis caused by infection due to fungal invasion into host tissues. The most common mycosis is classified into systemic mycosis or deep mycosis, subcutaneous mycosis, cutaneous mycosis, and superficial mycosis depending on the host infection route and site of infection.

Fungal infections have become a major health care problem worldwide over the past few decades as the number of immunocompromised patients due to AIDS, chemotherapy and organ transplantation increases. Despite this interest, the limited number of antifungal agents available for clinical use and the emergence of drug-resistant strains become a major obstacle in overcoming and treating various fungal infections caused by the genus *Candida, Aspergillus, Fusarium, Mucor* and *Cryptococcus.* In addition, since it is difficult to diagnose invasive fungal infections using conventional microbiological technologies and general imaging technologies, there is an urgent need to research and develop a diagnostic method for rapidly and accurately diagnosing invasive fungal infections. In this regard, Korean Patent No. 1799008 discloses a pharmaceutical composition for the treatment of fungal infections of keratinous tissues.

### TECHNICAL PROBLEMS

However, in the case of the prior art, there has been no research and development on the diagnosis of invasive fungal infection until now as the technology of the prior art relates to a composition for treating a fungal infection capable of improving drug permeation into keratinous tissue.

An object of the present invention is to provide a composition for diagnosing a fungal infection capable of rapidly and effectively diagnosing a fungal infection and acquiring a diagnostic image specialized for invasive fungal infection. However, these problems are exemplary, and the scope of the present invention is not limited thereto..

### TECHNICAL SOLUTIONS

According to one aspect of the present invention, there is provided a composition for diagnosing a fungal infection, containing 2-deoxy-2-[¹⁸F]fluorosorbitol ([¹⁸F]FDS) as an active ingredient.

According to another aspect of the present invention, there is provided a method for diagnosing a fungal infection, comprising administering an effective amount of [¹⁸F]FDS to a subject and obtaining a PET image.

### EFFECT OF THE INVENTION

As described above, by using the composition for diagnosing a fungal infection of the present invention, it is possible to non-invasively and relatively easily diagnose an invasive fungal infection, which has been difficult to diagnose using microbiological technologies and general imaging technologies. Of course, the scope of the present invention is not limited by these effects.

### BRIEF DESCRIPTION OF THE DRAWINGS

FIG. 1 is a graph showing the result of the radio TLC analysis of the primary labeled compound [¹⁸F]FDG (left) and the target labeled compound [¹⁸F]FDS (right) of the present invention.
FIG. 2 is a graph showing the result of the radio TLC analysis of a mixed sample including [¹⁸F]FDG and [¹⁸F]FDS.
FIG. 3 is a graph showing the analysis of the intake values of [¹⁸F]FDS for bacterial and fungal strains.
FIG. 4 is a microPET image showing the selective fungal uptake in the lung infection small animal model infected with *A. fumigatus* and administrated with [¹⁸F]FDS of the present invention.
FIG. 5 is a microPET image of a control group small animal model administrated with [¹⁸F]FDS of the present invention.
FIG. 6 is a microPET image of a muscle infection small animal model No. 1 infected with *A. fumigatus* and administrated with [¹⁸F]FDS of the present invention.
FIG. 7 is a microPET image of a muscle infection small animal model No. 2 infected with *A. fumigatus* and administrated with [¹⁸F]FDS of the present invention.

### DETAILED DESCRIPTION OF EMBODIMENTS

### Definition of Terms:

As used herein, "fungal infections" refers to infections caused by fungi, which are classified by the site of infection, particularly the depth. A superficial infection that invades the surface of the body, such as keratin or mucous membrane, can be classified into cutaneous infection and subcutaneous infection, and accounts for 90% of all mycosis. In addition, other systemic infections can be classified into systemic infections and opportunistic infections. Classification of fungal infections, the names of diseases and information on pathogenic fungi are summarized in Table 1 below.

**Table 1**

| Classification | Name of disease | Pathogenic fungi |
|---|---|---|
| Superficial infj ection | Pityriasis versicolor | *Malassezia furfur* |
| | Piedra | *Trichosporum beigelii* |
| | | *Piedraia hortae* |
| Cutaneous infection | Ringworm of scalp | *Epidermophyton* sp. |
| | glabrous skinnail | *Microsporum* sp. |
| | Candidiasis | *Trichophyton* sp. |
| | | *Candida albicans* etc. |
| Subcutaneous infection | Chromoblastomycosis | *Fonsecaea pedrosoi* |
| | Mycotic Mycetoma | *Pseudallescheria boydii* |
| | | *Madurella mycetomatis* |
| | Entomophthoromycosis | *Basidiobolus ranarum* |
| | | *Conidiobolus coronatus* |
| | Rhinosporidiosis | *Rhinosporidium seeberi* |
| | Lobomycosis | *Loboa loboi* |
| | Sporotrichosis | *Sporothrix schenckii* |
| Systemic infection | | |
| Systemic infection | Histoplasmosis | *Histoplasma capsulatum* |
| | Blastomycosis | *Blastomyces dermatitidis* |
| | Paracoccioidomycosis | *Paracoccidioides bransiliensis* |
| | Coccidioidomycosis | *Coccidioides immitis* |
| Opportunitic injfection | Cryptococcosis | *Cryptococcus neoformans* |
| | Aspergillosis | *Aspergillus fumigatus* etc. |
| | Mucormycosis | *Mucor* sp., *Absidia* sp., *Rhizopus* sp. |
| | Candidiasis (sytemic) | *Candida albicans* etc. |
| | Pneumocystis carinii | *Pneumocystis carinii* |
| | pneumonia | |
| Etc. | Phaeohyphomycosis | *Wangiella dermatitidis, Phialophora* sp. etc. |
| Rare mycosis | Hyalohyphomycosis | |
| | Basidiomycosis | *Schizophyllum commune* |

As used herein, "diagnosis" means identifying the presence or character of a pathological condition. For the purposes of the present invention, diagnosis is to determine whether a fungal infection has occurred.

### DETAILED DESCRIPTION OF THE INVENTION:

According to one aspect of the present invention, there is provided a composition for diagnosing a fungal infection, containing 2-deoxy-2-[¹⁸F]fluorosorbitol ([¹⁸F]FDS) as an active ingredient.

In the diagnostic composition, the fungus may be *Aspergillus fumigatus, Candida albicans* or *Rhizopus* sp., and the fungal infection may be systemic infection or opportunistic infection. In this case, the systemic infection may be Histoplasmosis, Blastomycosis, Paracoccidioidomycosis, or Coccidioidomycosis, and the opportunistic infection may be Cryptococcosis, Aspergillosis, Mucormycosis, Candidiasis or Pneumocystis carinii pneumonia.

According to another aspect of the present invention, there is provided a method for diagnosing a fungal infection, comprising administering an effective amount of [¹⁸F]FDS to a subject and obtaining a PET image. In this case, the subject may be a mammal other than a human.

Also provided is a contrast agent for positron emission tomography (PET) comprising the composition for diagnosing a fungal infection of the present invention as an active ingredient. The contrast agent for PET may be combined with a pharmaceutically acceptable carrier according to conventional pharmaceutical preparation technologies. Such carriers may take a wide variety of forms depending upon the preparation desired, for example, for oral or parenteral (including intravenous) administration. In addition, the contrast agent may be administered in a dose of 0.1 mg/kg to 1 g/kg, more preferably 0.1 mg/kg to 500 mg/kg. On the other hand, the dosage may be appropriately adjusted according to the age, sex, and condition of the patient within the range of daily or annual allowable radiation exposure.

In addition, the contrast agent further includes an inert component including a pharmaceutically acceptable carrier. As used herein, "pharmaceutically acceptable carrier" is a term that refers to components other than the active substance of a composition, specifically, a pharmaceutical composition. Examples of pharmaceutically acceptable carriers include binders, disintegrants, diluents, fillers, glidants, solubilizers or emulsifiers and salts. The contrast agent may be administered to the subject by parenteral administration, and the parenteral administration includes intravenous injection, intraperitoneal injection, intramuscular injection, or subcutaneous administration, but intravenous administration is most preferred.

Invasive fungal infections previously reported have been difficult to diagnose with microbiological technologies and general imaging technologies, but there has been no research and development on them. The present inventors have made intensive efforts to solve the above problems, and as a result, developed a composition for diagnosing a fungal infection that can obtain a diagnostic image specialized for invasive fungal infection using the radioactive tracer 2-deoxy-2-[¹⁸F]fluorosorbitol ([¹⁸F]FDS). Using the composition of the present invention, invasive fungal infections, which have been difficult to diagnose with conventional microbiological technologies and general imaging technologies, can be diagnosed non-invasively and relatively easily. In particular, it has the advantage of being able to differentiate fungal infections from non-specific inflammation or bacterial infections (except for Enterobacteriacae). Since Enterobactriacae utilizes [¹⁸F]FDS like fungi, although the possibility that it can be used as an image tracer for diagnosing human infections of Enterobacteriacea is suggested, but the present inventors have confirmed that the degree of uptake of [¹⁸F]FDS by fungi is more than 10 times higher than bacterial uptake in the in vitro experiment. Thus, if the above invention is actually used for diagnosis of fungi, it is expected that fungal infections can be differentiated from bacterial infections according to uptake level of [¹⁸F]FDS like the usage of [¹⁸F]FDG-PET in the diagnosis of cancers. In addition, considering that Enterobacteriaceae mainly causes intra-abdominal infections, while invasive fungal infections mainly cause problems in the lungs and brains, and the patients suffering from invasive fungal infection are also mainly limited to immunocompromised persons, it is believed that the composition for diagnosing fungal infection can secure diagnostic specificity for invasive fungal infection.

In addition, if an invasive fungal infection of the human body is diagnosed at an appropriate time through a specialized diagnostic image, the patient's prognosis
can be improved by using an appropriate antifungal agent, and the amount of empirical antifungal agent used because a definitive diagnosis cannot be made can be reduced. Therefore, it is possible to solve problems such as reducing medical costs
and increasing resistance to antifungal drugs. There is still no technology for a diagnostic composition capable of accurately diagnosing various fungal infections.

### Best Mode for Carrying Out the Invention

Hereinafter, preferred embodiments of the present disclosure will be described in detail. However, the present disclosure is not limited to embodiments explained herein but may be specified in various aspects. Rather, the embodiments are provided to sufficiently transfer the concept of the present disclosure to a person skilled in the art to thorough and complete contents introduced herein.

### Example 1: Synthesis of composition for diagnosis

The present inventors synthesized the radioactive tracer of the present invention, 2-deoxy-2-[¹⁸F]fluorosorbitol ([¹⁸F]FDS). To this end, radioactive fluorine (¹⁸F) was first produced by a reaction of ¹⁸O(p,n)¹⁸F using a cyclotron. The produced ¹⁸F 1-3 Ci was adsorbed using an anion exchange resin cartridge, and then eluted using potassium carbonate and Kryptofix 2.2.2 solution. After the drying process to impart chemical activity of the eluted ¹⁸F, it was reacted with mannose triflate, a precursor of 2-deoxy-2-[¹⁸F]fluoro-D-glucose([¹⁸F]FDG) at 30°C for 5 minutes. Thereafter, a deprotection process for the hydroxyl functional group was performed using sodium hydroxide, and [¹⁸F]FDG, the primary target radioactive compound, was isolated using a disposable separation cartridge. Then, 2 mg of sodium borohydride (NaBH₄) was added to the separated [¹⁸F]FDG and reduced by stirring at 40°C for 15 minutes. Finally, to separate [¹⁸F]FDS, the final target radioactive compound, the reactant was separated using an Alumina N cartridge and eluted with 1 mL of physiological saline solution. For biological evaluation, the pH was adjusted to neutral, passed through a sterile filter, and prepared in a sterile vial.

### Example 2: Fungal cultivation

The present inventors cultured the fungus using the following method. Specifically, *Aspergillus fumigatus* was smeared on potato dextrose agar medium (or YAG, yeast extract agar medium), and cultured at 30°C for 2-3 days. Thereafter, the culture plate was washed with sterile physiological saline and Tween 20 [10 µl (1 drop)] and filtered with sterile gauze (or 40 µm-pore insertion filter). Then, the pellet was recovered using a centrifuge and the number of fungi was confirmed to prepare a bacterial solution of 1.5×10⁹ conida/mL. *Rhizopus arrhizus* isolated from the patient was also cultured in the same way as *A. fumigatus,* prepared a fungal solution, and used for *in vitro* experiments. For *Candida albicans,* a strain isolated from the patient was also used, and cultured in SDA medium for 2-3 days. After recovering from the medium, a fungal solution was prepared.

### Example 3: In vitro analysis

The present inventors performed *in vitro* experiments on the strain cultured in Example 2 above. Specifically, each strain was prepared to be 1×10⁸ per 1 mL of medium, and 1 mL was dispensed into tubes. The experiments were performed three times according to the experimental period, and 0.74 MBq of [¹⁸F]FDS synthesized as described above was injected and incubated for 2 hours in the incubator. After 2 hours, each strain was divided into a filterable 1.5 mL centrifuge tube, and the fungi and medium were separated through centrifugation. After washing twice with phosphate buffer, the filtered fungi were collected and the intake was measured with a gamma counter.

### Example 4: Preparing a lung infection model

The present inventors created a lung infection model by the following method. First, after preparing Balb/c mice 8 weeks old (female), 150 mg/kg 100 µl of cyclophosphamide was intraperitoneally injected 4 days before infection. After 3 days, 100 µl of 250 mg/kg of cortisone acetate was injected into the subcutaneous fat layer, and the mice were anesthetized after 24 hours. And then the lung infection model mice were prepared by the injection of 70 µl of *A. fumigatus* fungal solution (about 1.5×10⁷ conidia) through the nasal cavity.

### Example 5: Preparing a muscle infection model

The present inventors produced a muscle infection model by the following method. First, after preparing Balb/c mice 8 weeks old (female), 150 mg/kg 100 µl of cyclophosphamide was intraperitoneally injected 4 days before infection. After 3 days, 100 µl of 250 mg/kg of cortisone acetate was injected into the subcutaneous fat layer, and the mice were anesthetized after 24 hours. And then the muscle infection model mice were prepared by the injection of 100 µl of *A. fumigatus* fungal solution (about 1.5×10⁸ conidia) into the forelimb muscle of the mice.

### Example 6: MicroPET image acquisition

The present inventors acquired microPET images 2 hours after injecting the [¹⁸F]FDS (7.4 MBq) according to the present invention into the tail vein of the infected mice, and the acquiered images were reconstructed by using the 3D-ordered subset expectation maximization (3D-OSEM) method.

### Experimental Example 1: Identification of [¹⁸F] FDS

After the labeling reaction to confirm the [¹⁸F]FDS of the present invention, the present inventors used a radio TLC scanner (TLC development conditions acetonitrile:water = 4:1) to determine the primary radioactive compound, [¹⁸F]FDG, and the final radioactive compound, [¹⁸F]FDS, in order to identify whether [¹⁸F]FDS was isolated and to check the purity of [¹⁸F]FDS.

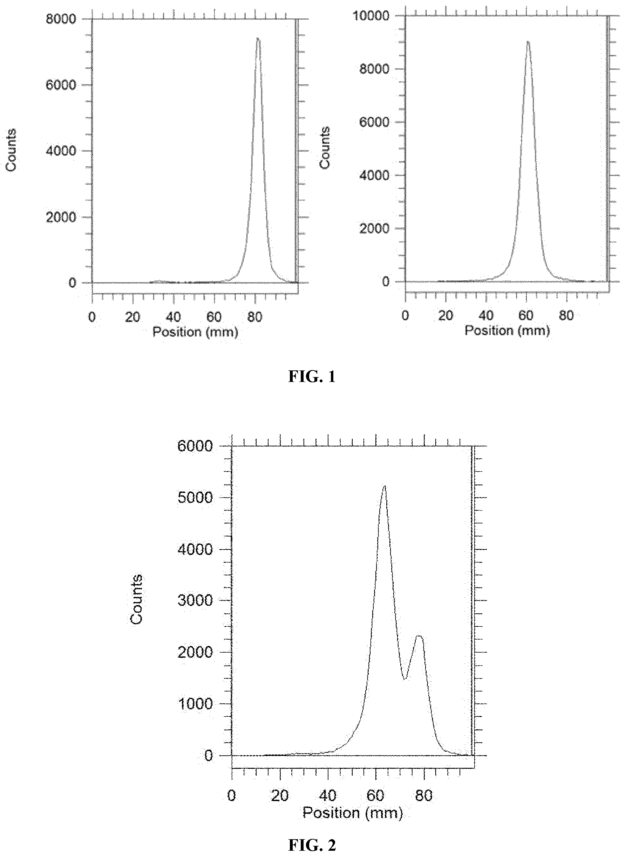

As a result, the primary labeled radioactive compound [¹⁸F]FDG (left) and the final target labeled radioactive compound [¹⁸F]FDS (right) showed different Rf values (FIG. 1). This proved that 100% [¹⁸F]FDS was reduced in [¹⁸F]FDG when comparing each result. Finally, radio TLC analysis of samples mixed with [¹⁸F]FDG and [¹⁸F]FDS was performed and two separated peaks were identified (FIG. 2).

### Experimental Example 2: Analysis of intake ability of [¹⁸F]FDS

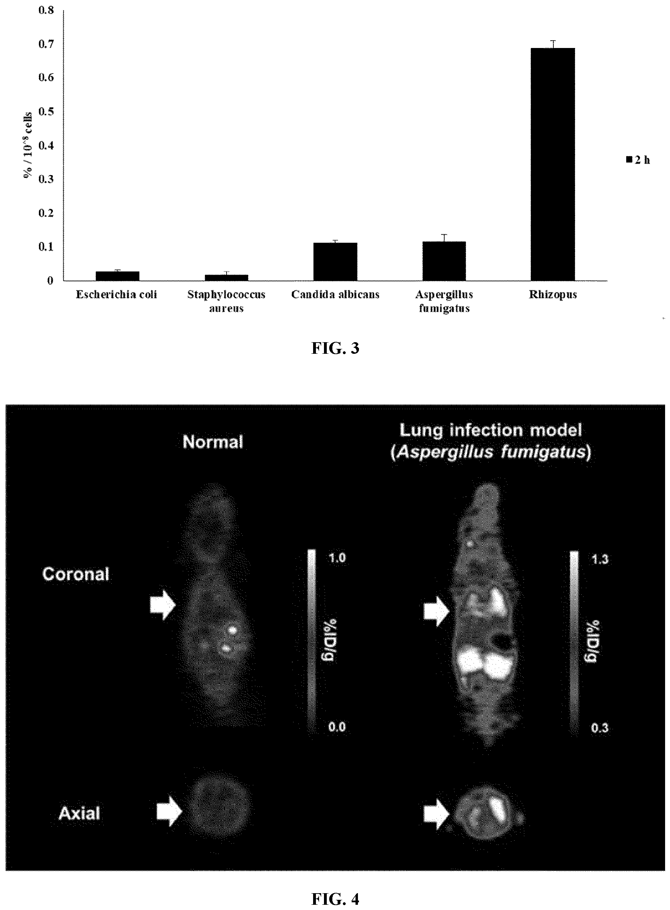

The present inventors confirmed the uptake ability of [¹⁸F]FDS of the present invention using fungal strain cultivation. It was confirmed that gram-positive bacterial strains did not show no uptake of [¹⁸F]FDS and only gram-negative bacterial strains showed uptake of [¹⁸F]FDS. However, in the present invention, it was confirmed that not only the Gram-negative strains but also the fungal strains such as *C. albicans, A. fumigatus* and *Rhizopus* sp. showed a very high selective uptake of [¹⁸F]FDS (FIG. 3).

### Experimental Example 3: Small animal infection model experiment

The present inventors confirmed the fungal selective uptake ability of [¹⁸F]FDS of the present invention using small animal infection models. To this end, [¹⁸F]FDS was administered intravenously to Balb/c mice infected with *A. fumigatus* in the lung prepared in Example 4, and microPET images were acquired and analyzed. As a result, very high selective uptake of [¹⁸F]FDS in the lungs was confirmed, and the same results were obtained *in vitro* experiments (FIG. 4).

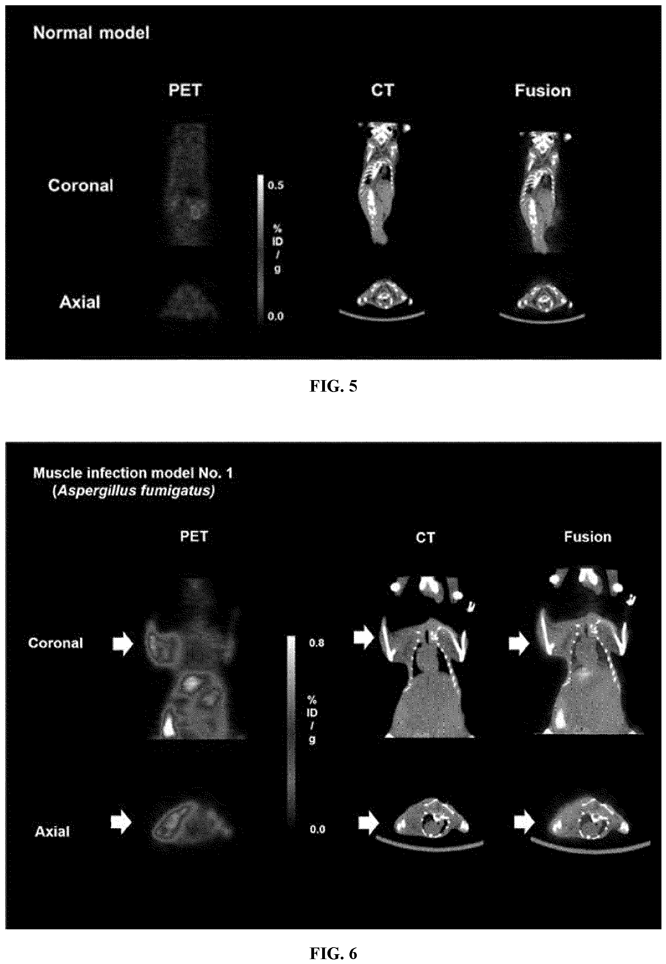

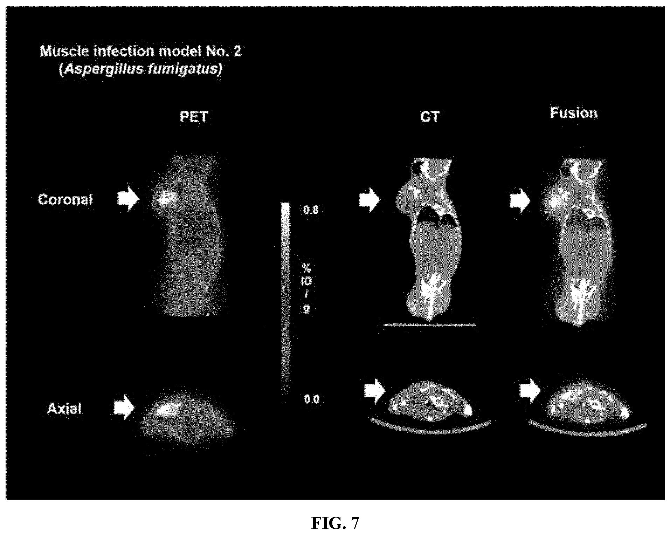

In addition, as a result of confirming [¹⁸F]FDS uptake using the *A. fumigatus* muscle infection small animal model prepared in Example 5, a very high selective uptake of [¹⁸F]FDS at the muscle infection site compared to the control group (FIG. 5) was confirmed (FIGs. 6 and 7). The experimental results of the lung and muscle infection model suggest that fungal infections and pathogenic fungal strains can be quickly and accurately diagnosed using the [¹⁸F]FDS of the present invention.

In conclusion, since [¹⁸F]FDS of the present invention is a substance that has already been shown to have potential for use in intestinal bacteria, infection diagnostic images, and kidney function evaluation images in the human body and showed a high selective uptake ability in the lung infection model and the muscle infection model infected with fungi as described above, it can be used clinically as a rapid and efficient contrast agent for diagnosing fungal infections.

While the present invention has been described with reference to exemplary examples and experimental examples, it is to be understood that the invention is not limited to the disclosed exemplary examples and experimental examples, but, on the contrary, is intended to cover various modifications and equivalent arrangements included within the spirit and scope of the appended claims. Accordingly, the true scope of the present invention should be determined by the technical idea of the appended claims.

## Claims

1. A composition for diagnosing a fungal infection, comprising 2-deoxy-2-[¹⁸F]fluorosorbitol ([¹⁸F]FDS) as an active ingredient.

2. The composition according to claim 1, the fungal infection is caused by *Aspergillus fumigatus, Candida albicans* or *Rhizopus* sp.

3. The composition according to claim 1, wherein the fungal infection is a systemic infection or an opportunistic infection.

4. The composition according to claim 3, the systemic infection is Histoplasmosis, Blastomycosis, Paracoccidioidomycosis, or Coccidioidomycosis and the opportunistic infection is Cryptococcosis, Aspergillosis, Mucormycosis, Candidiasis, or Pneumocystis carinii pneumonia.

5. A method for diagnosing a fungal infection in a subject, comprising administering an effective amount of [¹⁸F]FDS to the subject; and obtaining a PET image.
